# EUROPEAN PATENT SPECIFICATION

(11) **EP 0 494 158 B1**
(45) Date of publication and mention of the grant of the patent: **10.03.1999**
(21) Application number: 90912568.4
(22) Date of filing: 31.08.1990
(51) Int. Cl.: C12N 15/16, C12N 15/36, C12N 15/86, A61K 39/29

(54) **A RECOMBINANT BIRTH CONTROL VACCINE**
REKOMBINANTER IMPFSTOFF ZUR GEBURTENKONTROLLE
VACCIN REBOMBINANT DE LIMITATION DES NAISSANCES

(30) Priority: 29.09.1989 CA 614520
(43) Date of publication of application: 15.07.1992
(73) Proprietor: NATIONAL INSTITUTE OF IMMUNOLOGY, New Delhi 11067 (IN)
(72) Inventor: TALWAR, Gursaran, Prasad, Shalid Jeet Singh Marg New Delhi (IN); SRINIVASAN, Jay, Shahid Jeet Singh Marg New Delhi (IN); CHAKRABARTI, Sekhar, Room 237, Building 4 Bethesda, MD 20892 (US)
(74) Representative: Davies, Jonathan Mark
(86) International application number: CA9000276
(87) International publication number: WO9105049

(56) References cited:
- EP-A- 204 566
- WO-A-86/07383
- JOURNAL OF VIROLOGY vol. 62, no. 10, October 1988, AMERICAN SOCIETY FOR MICROBIOLOGY pages 3772 - 3778; NILES, E.G. & SETO, J.: "Vaccinia virus gene D8 encodes a virion transmembrane protein."
- GENE. vol. 77, no. 1, 15 April 1989, AMSTERDAM NL pages 87 - 93; CHAKRABARTI, S. et al.: "Expression of biologically active human chorionic gonadotropin and its subunits by recombinant vaccinia virus." (cited in the application)

## Description

Population is growing at a rapid pace in many . economically developing countries and there is a continuing need of an alternate method for regulation of fertility. We proposed several years back a birth control vaccine which induces the formation of antibodies against the human pregnancy hormone, the human chorionic gonadotropin (hCG). These inventions are described in patents issued in India, U.S.A. and several other countries. (Ref. EP 204566, JP 62286928, CA 1239346, US 4780312, CN 8603854). We describe now another invention which generates antibody response of a long duration against hCG after a single or a limited number of injections.

Whereas the possibility of controlling fertility by raising antibodies against hCG is known from our previous studies and those of others, the vaccines utilized earlier were conjugates of two or more peptides such as the natural beta hCG peptide of 145 amino acids linked to tetanus toxoid or other carriers. In another modality, the beta hCG peptide was associated with alpha oLH and then linked to carriers (Talwar et al 1988; US Patent No. 4,780,312). These vaccines demand purification and preparation of the constituent proteins from natural sources. The cost of some of these is at present very high which will be restrictive to their large scale use in family planning programs of economically developing countries. Moreover these vaccines demand three injections for primary immunization followed by a fourth as booster. A major advantage of the present embodiment is the possibility of getting satisfactory and sustained antibody response with one primary injection and at most one booster. Another interesting feature is the low cost at which this vaccine can be prepared and made available for large scale use.

Vaccinia virus is well known as a versatile tool for molecular biologists. In the New Scientist dated 3 December 1988 (Anon, p.38) an article refers to a new vaccine for rinderpest virus in cattle and states that the vaccine is a genetically engineered version of the vaccinia virus, researchers having transformed two genes coding for the coating of rinderpest virus into the vaccinia virus.

In a Tibtech article dated January 1990, Miner et al (p. 20-25), discusses vaccinia virus as a vesatile tool for molecular biologists and suggests that "the vaccinia virus system is a promising way of producing significant amounts of correctly processed and modified eukaryotic proteins in mammalian cells".

U.S. Patent 4,603,112 (inventors: E. Paoletti et al; issued: July 29th, 1986) discloses methods for modifying the genome of vaccinia virus to produce recombinant vaccinia virus comprising DNA not naturally occurring in vaccinia virus. While this reference includes reference to the heterologous DNA coding for a protein which may be an antigen, it does not appear to make any suggestion that this technology is a route to birth control vaccines or birth control vaccines which also double-up as vaccines capable of raising antibodies against proteins or peptides unassociated with the mammalian reproductive system.

PCT application number US88/00614 (inventors: B.R. Bloom et al; filed: 29 February 1988) relates to the use of recombinant mycobacteria as vehicles capable of expressing foreign DNA. It also indicates that such mycobacteria could be used as an anti-fertility vaccine vehicle. However, it appears to make no claim to the use of mycobacteria for control of fertility and also directs readers away from the use of vaccinia in this field pointing out alleged disadvantages on pages 4 and 5.

In an article in Gene, 77 (1989) 87-93, Chakrabarti et al discussed the expression of biologically active human chorionic gonadotropin and its subunits by recombinant vaccinia virus. Vaccinia virus expression vector was used to clone the genes for coding alpha and beta subunits of human chorionic gonadotropin. Recombinant viruses containing these genes were selected. Cells infected with the recombinant viruses secreted alpha and beta hCG subunits. The subunit proteins expressed individually had immunoreactivity with monoclonal and polyclonal antibodies specific to hCG.

PCT publication No. WO 86/07383 discloses autoantigen vaccines which are obtained by conferring antigenicity by formation of multimers of fusion proteins to non-bacterial sequences. A vaccine for preventing human pregnancy is said to have been produced which comprised a vaccine virus vector modified to contain the DNA sequence encoding the C-terminal portion of the beta chain of human chorionic gonadotropin. The immunogenicity of the C-terminal portion of the beta chain of hCG may be enhanced by inserting it into the antigenic regions of a protein derived from an infectious material such as a viral surface protein, or to a "carrier" additional peptide of sufficient size to cause the raising of antibodies against it: in the Examples the peptide sequence of influenza haemagglutonin protein is said to be particularly useful in providing the needed additional amino acid sequence.

There appears to be a need to provide a safe, simple and effective birth control vaccine at low cost which can also, if required, act as a vaccine against non-reproductive-system-associated disorders such as infections by bacteria and viruses. While some of the references suggest that the use of vaccinia as a biotechnological tool, other references, especially Bloom et al appear strongly to discourage consideration of vaccinia. In view of the expenses involved in this type of research it therefore cannot be said that there are clear signposts to research works in this area pointing to the use of vaccinia in birth control vaccines.

The present invention provides a nucleotide sequence (I) coding for a fused peptide, the sequence consisting of
(A) a beta subunit of a mammalian gonadotropin in reading frame alignment with and followed by
(B) the trans-membrane and cytoplasmic domains of the gene coding for Vesicular Stomatitis Virus Glycoprotein.
whereby when inserted into a virus and the virus is used to infect a host cell, the fused peptide is expressed and anchored to the host cell membrane.

The invention also provides a nucleotide sequence (I) referred to above which further includes a sequence (II) coding for an alpha subunit of ovine luteinizing hormone inserted into a viral nucleotide sequence and capable of binding to the membrane anchored peptide on co-expression with the membrane anchored peptide in the same host cell.

The above nucleotide sequences are preferably inserted into a nonessential part of a vaccinia virus genome to give a recombinant vaccinia virus.

The invention also provides a recombinant vaccinia virus consisting of the nucleotide sequences referred to above inserted into a region of the vaccinia virus genome non-essential for survival of vaccinia virus in a host cell.

The invention also provides a birth control vaccine comprising at least one recombinant virus as described above.

The invention also provides a pharmaceutical composition comprising a nucleotide sequence as described above.

The invention also provides a vaccine for raising antibodies against a beta subunit of chorionic gonadotropin comprising a pharmaceutical composition as described above.

In drawings which illustrate embodiments of the invention;
Figure 1 shows the strategy for the construction of anchored beta hCG;
Figure 2 shows the strategy for the insertion of alpha oLH gene into vaccinia virus; and,
Figure 3 demonstrates the anti hCG response in terms of antigen binding capacity. The figure shows typical antibody response in four rats given a single injection of vSS2 recombinant vaccine at a dose of 10 pfu (plaque forming units). Each animal response with antibodies in circulation measurable at the end of four weeks. The titers ranged from 100 to 900 ng/ml and are distinctly above the threshold value of 20 ng/ml considered to be protective against pregnancy. The titers were sustained over 12 weeks of observation.
Figures 4a to 4h show development of hCG antibodies in 8 bonnet monkeys injected with vSS2 recombinant vaccine.

### EXAMPLE 1 SYNTHESIS AND UTILITY OF RECOMBINANT ANTI-hCG VACCINE

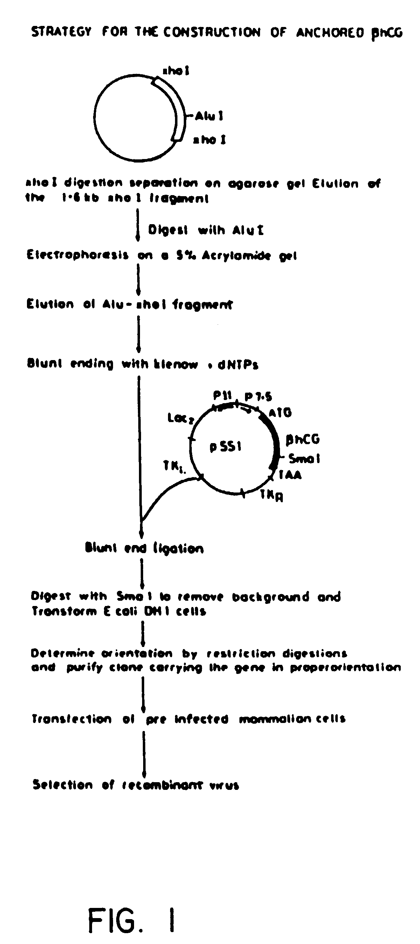

The construction strategy of vSS2: The virus containing the anchored beta hCG was made by an in-frame fusion of the trans- membrane and cytoplasmic domains of the gene coding for vesicular stomatitis virus glycoprotein (VSVg) to the 3 ' end of the beta hCG cDNA (Fig. 1). The VSVg gene was digested with Alu I and Xho I to release the 249 bp membrane anchor sequence. This fragment was eluted out from an acrylamide gel, klenow filled and ligated to the Sma I cut vector pSS I, the latter was prepared by the procedure described by us previously (Chakrabarti et al 1989). Orientation of the anchor sequence with reference to beta hCG was checked by suitable restriction enzyme digestions. This was used to transfect CV-1 cells preinfected with wild type virus and the recombinants were picked up by visual screening for the blue plaques by the technique described elsewhere.

Assay for beta hCG: The expression of beta hCG was detected in the pellet of the vSS2 infected cells by a competitive radio-immunoassay using mouse monoclonal antibodies raised against beta hCG. The cellular localisation of hCG was determined by immunofluorescence technique using anti beta hCG MoAb followed by rabbit anti mouse conjugated to FITC.

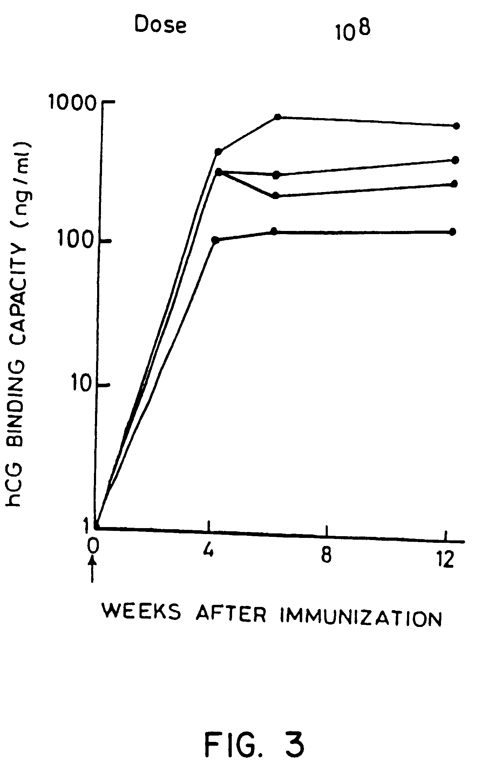

**Immunogenic properties:** A single intra-dermal injection of 10⁸ pfu of vSS2 elicited the formation of antibodies in rats which reacted with hCG (Fig. 3) and prevented effectively the binding of hCG to receptors on target tissue. The antibodies were detectable within four weeks. The titers were sustained without a decline over a period of several months.

Antibody titers in monkeys immunized with recombinant anti hCG vaccine (vSS2):

Bonnet monkeys (Mecacca radiata) were immunized intradermally with 10⁸ pfu of the recombinant vaccine. Two immunizations were done at 3 months interval (day 0 to 95). Four months later (day 226), a booster injection of 100 ug of beta hCG adsorbed on alum was given intramuscularly. Anti hCG antibodies were measured (□―□) by radio-immunoassay (see Om Singh et al). These are expressed on the ordinate on a logarithmic scale. The antibody titers were measurable after the first immunization with the recombinant vaccine. Their titers after two primary and a booster injection increased to very high levels ranging from 3,200 to 14,000 ng of hCG binding capacity per ml. These antibodies had high affinity (ka = 10⁻¹¹LM). The antibodies were competent to prevent the binding of hCG to the target tissue receptors as determined by competitive radio receptor assays (+―+). The competence of the antibodies in neutralizing the bioefficacy of hCG indicates the efficacy of immunization with such vaccines in order to intercept events supported by hCG, such as the establishment and sustenance of early pregnancy.

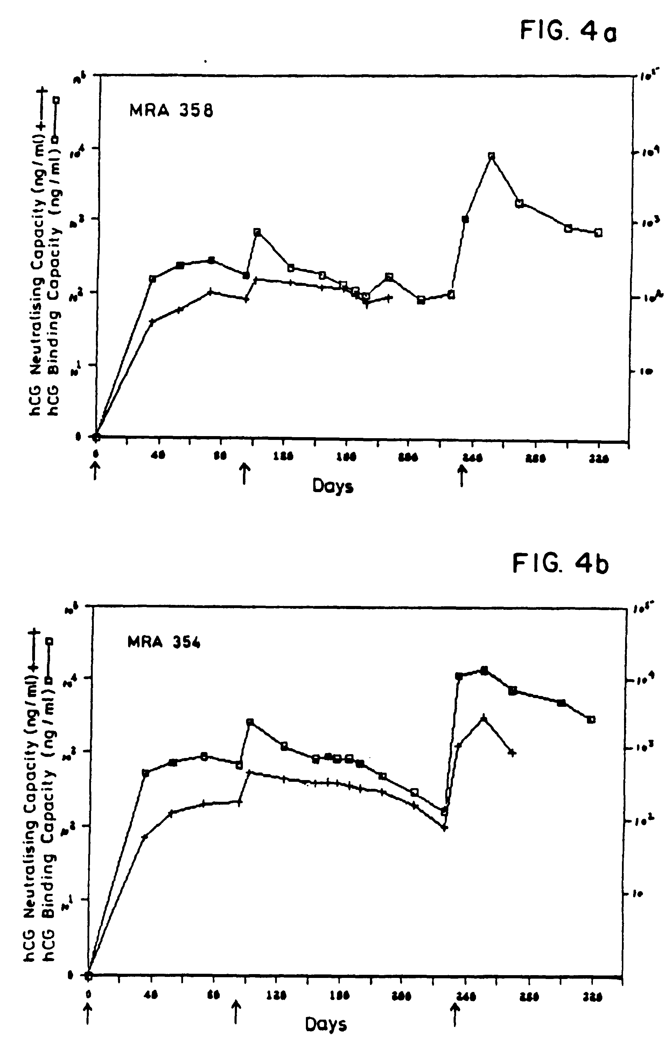

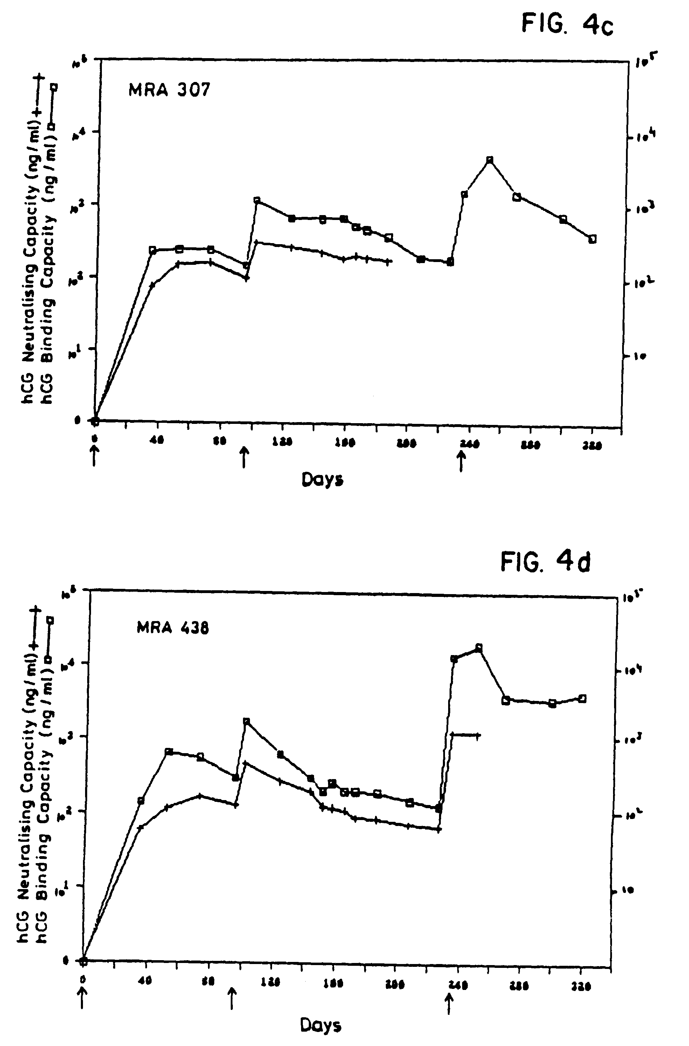

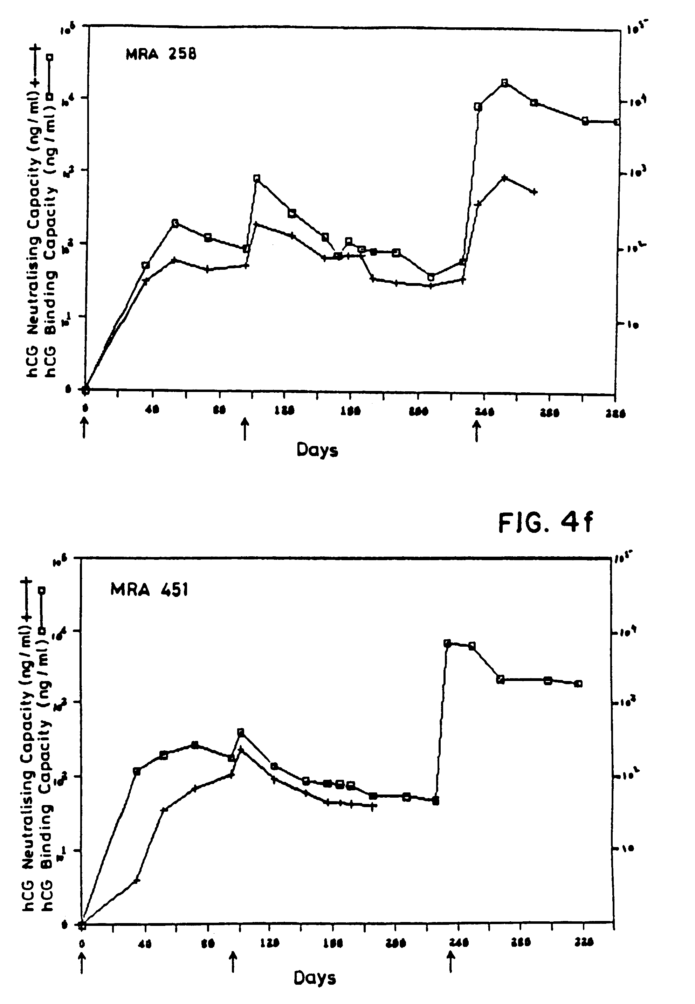

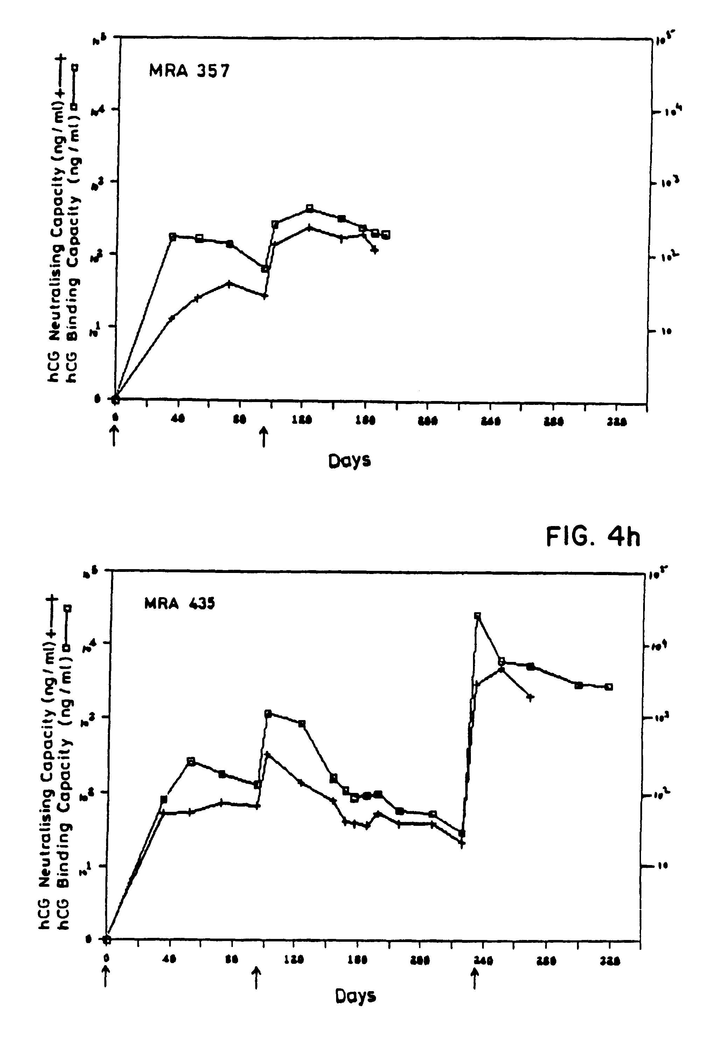

Figure 4 gives data in eight monkeys to demonstrate the consistency of the phenomenon. These experiments also demonstrate the immunogenicity of these products not only in rodents but also in primates (and, by extension, humans).

To date, the best results in both monkeys and rats have been obtained with 10⁸pfu. An operating range runs from about 10⁹ down to about 10⁴pfu preferably of the order of 10⁷ to 10⁸pfu. Interestingly, dose does not appear to work in this case on a body weight basis and this may be related to the use of live vaccine. Attenuation by passaging may be useful to avoid possible side effects.

### Example 2: Synthesis and utility of anti-oLH vaccine (NOT ACCORDING TO THE INVENTION)

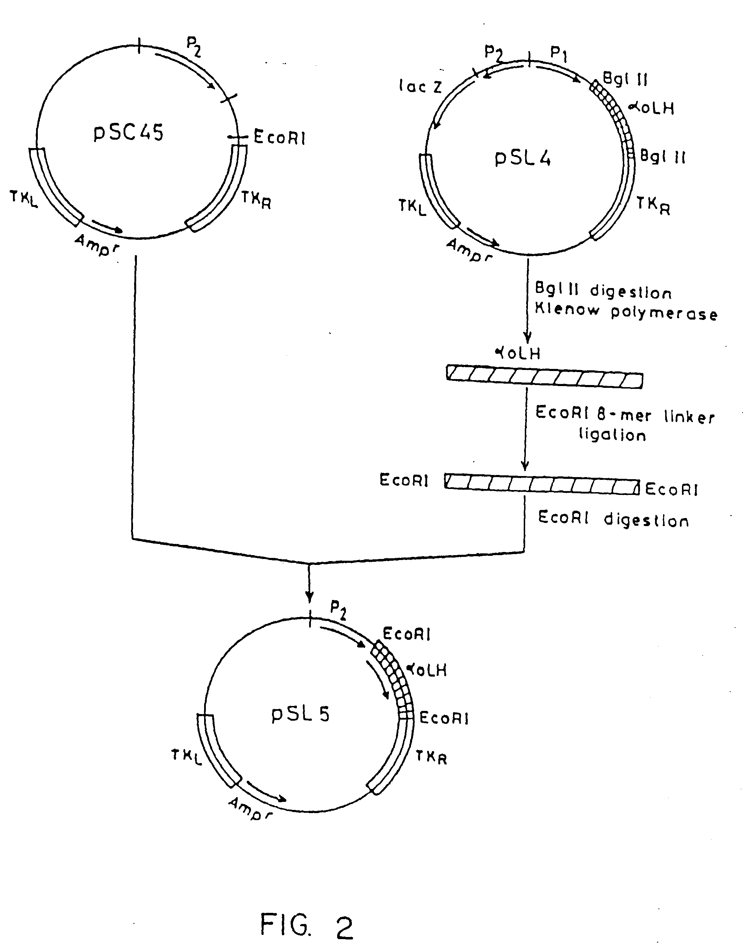

Construction of vSL5. The alpha oLH cDNA was cut out by the restriction enzyme Bgl II from a previously described vector (Lall et al 1988). This fragment was klenow filled, ligated to 8-mer Eco RI linkers and cloned into the unique Eco RI site of the vaccinia vector pSC45 (Fig. 2). The final plasmid, pSL5, was characterised in detail with respect to the correct orientation of the alpha oLH gene by multiple restriction enzyme digestions and Southern hybridization. This plasmid was subsequently used in DNA transfection to construct the recombinant virus vSL5.

Assay for alpha oLH: The medium of the cells infected with recombinant virus vSL5, was assayed for the presence of alpha oLH by a competitive radio-immunoassay using anti alpha oLH antibodies raised in monkeys. The expression of alpha oLH was quantitated using a standard subunit peptide. The presence of alpha oLH could be detected within three hours of infection and was found to be 280ng/ml/3x10⁶ cells in 24 hours.

### Example 3: Production of mixed recombinant vaccines

Biological activity: The alpha oLH subunit associates with beta hCG to form a hetero-dimer. The ability of this hetero-dimer to stimulate steroidogenesis in a Leydig cell system is well established (Talwar et al., 1988). In order to show that the recombinant alpha oLH-beta hCG hetero-dimer retains its biological activity, a co-infection of viruses vSS1 and vSL5 was done in the CV1 cells and the supernatant, collected 24h post-infection was used in a Leydig cell bio-assay. The steroidogenesis elicited by the vaccinia expressed alpha oLH-beta hCG hetero-dimer was greater than the native hCG dimer indicating the correct and full length expression of the two peptides.

A similar and somewhat bio-effectively better immune-response to that shown by construct vSS2 alone could be generated by using a mixture of the constructs vSS2 and vSL5. It is known from other studies that the antibodies generated by a heterospecies dimer of beta hCG and alpha oLH have about 25% better bio-efficacy as a function of their immunological titers as compared to those generated to beta hCG alone.

The new live recombinant vaccines described here were well tolerated, no side effects were observed during standard acute and subacute toxicology studies in the two animal species studied to date. These vaccines can be employed with conventional pharmaceutically acceptable diluents.

### REFERENCES NOT DETAILED IN THE DISCLOSURE

S. Chakrabarti, Srinivasan. J, L. Lall, L.V. Rao and G.P. Talwar: Expression of biologically active human chorionic gonadotropin and its subunits by recombinant vaccinia virus Gene, 77, (1989) 87-93.

Chakrabarti S, Brechling K and Moss B.: Vaccinia virus expression vector: Co-expression of B-galactosidase provides visual screening of recombinant viral plagues. Mol. Cell. Biol., 5, (1985) 3403-3409.

Jain S.K, Chin W.W and Talwar. G.P.: Isolation and characterization of cDNA clones for and B subunits of ovine luteinizing hormone. J. Biosci., 12, (1987) 349-357.

Lavanya Lall, J. Srinivasan, L.V. Rao, S.K. Jain, G.P. Talwar and S. Chakrabarti.: Recombinant vaccinia virus expresses immunoreactive alpha subunit of Ovine Luteinizing Hormone which associates with B-hCG to generate bioactive dimer. Indian J. Biochem. Biophy., 25, (1988) 510-514.

Talwar G.P, Om Singh and Rao L.V.: An improved immunogen for anti-hCG vaccine eliciting antibodies reactive with conformation native to the hormone without cross-reaction with hFSH and hTSH. J. Repro. Immu., 13, (1988) 53-63.

Om Singh, N.C. Sharma, L.V, Rao, A. Alam A. Gaur and G.P. Talwar (1989). Antibody response and characteristics of antibodies in women immunized with three contraceptive vaccines inducing antibodies against human chorionic gonadotropin. Fertility and Sterility: vol. 52, No. 5, 739-744.

## Claims

1. A nucleotide sequence (I) coding for a fused peptide, the sequence consisting of
(A) a beta subunit of a mammalian gonadotropin in reading frame alignment with and followed by
(B) the trans-membrane and cytoplasmic domains of the gene coding for Vesicular Stomatitis Virus Glycoprotein.
whereby when inserted into a virus and the virus is used to infect a host cell, the fused peptide is expressed and anchored to the host cell membrane.

2. A nucleotide sequence (I) according to claim 1 which further includes a sequence (II) coding for an alpha subunit of ovine luteinizing hormone inserted into a viral nucleotide sequence and capable of binding to the membrane anchored peptide on co-expression with the membrane anchored peptide in the same host cell.

3. A recombinant vaccinia virus consisting of a nucleotide sequence according to claim 1 or 2 inserted into a region of the vaccinia virus genome non-essential for survival of vaccinia virus in a host cell.

4. A birth control vaccine comprising at least one recombinant virus according to claim 3.

5. A pharmaceutical composition comprising a nucleotide sequence according to claim 1 or 2.

6. A vaccine for raising antibodies against a beta subunit of chorionic gonadotropin comprising a pharmaceutical composition according to claim 5.

## Patentansprüche

1. Für ein Fusionspeptid codierende Nucleotidsequenz (1), wobei die Sequenz besteht aus
(a) einer beta-Untereinheit eines Gonadotropins eines Säugers in Lese-Raster-Ausrichtung mit und gefolgt von
(b) der Trans-Membran und den Cytoplasmabereichen des Gens, welches für das Glycoprotein des vesikularen Stomatitisvirus (vesicular stomatitis virus glycoprotein, VSVg) codiert,
wobei das Fusionspeptid bei Einführung in einen Virus, der zum Infizieren einer Wirtszelle verwendet wird, exprimiert und an der Membran der Wirtszelle verankert wird.

2. Nucleotidsequenz (I) gemäß Anspruch 1, welche weiterhin eine Sequenz (II) aufweist, die für eine alpha-Untereinheit des Ei-Luteinisierungshormons codiert, welches in eine virale Nucleotidsequenz eingeführt ist und befähigt ist, sich an mit der Membran verankertes Peptid zu binden, wenn die Co-Expression mit dem mit der Membran verankerten Peptid der gleichen Wirtszelle erfolgt.

3. Rekombinanter Vacciniavirus, bestehend aus einer Nucleotidsequenz gemäß Anspruch 1 oder 2, die in einen Bereich des Genoms des Vacciniavirus eingeführt ist, der für das Überleben des Vacciniavirus in einer Wirtszelle nicht essentiell ist.

4. Impfstoff zur Geburtenkontrolle, aufweisend mindestens einen rekombinanten Virus gemäß Anspruch 3.

5. Pharmazeutische Zusammensetzung, enthaltend eine Nucleotidsequenz gemäß Anspruch 1 oder 2.

6. Impfstoff zum Züchten von Antikörpern gegen eine beta-Untereinheit des Choriongonadotropins, enthaltend eine pharmazeutische Zusammensetzung gemäß Anspruch 5.

## Revendications

1. Séquence nucléotidique (I) codant pour un peptide fusionné, la séquence consistant en
a) une sous-unité β d'une gonadotropine de mammifère dans un alignement en phase de lecture avec et suivi par
b) les domaines trans-membranaires et cytoplasmiques du gène codant pour une glycoprotéine du virus de la stomatite vésiculeuse,
au moyen de laquelle, lorsqu'elle est insérée dans un virus et que le virus est utilisé pour infecter une cellule hôte, le peptide fusionné est exprimé et est ancré à la membrane de la cellule hôte.

2. Séquence nucléotidique (I) selon la revendication 1 qui comporte en outre une séquence (II) codant pour une sous-unité α d'une hormone lutéinisante ovine insérée dans une séquence nucléotidique virale et capable de se lier au peptide ancré dans la membrane lorsqu'elle est co-exprimée avec le peptide ancré dans la membrane dans la même cellule hôte.

3. Virus recombinant de la vaccine qui consiste en une séquence nucléotidique selon la revendication 1 ou 2 insérée dans une région du génôme du virus de la vaccine non essentielle pour la survie du virus de la vaccine dans la cellule hôte.

4. Vaccin pour le contrôle de la naissance comprenant au moins un virus recombinant selon la revendication 3.

5. Composition pharmaceutique comprenant une séquence nucléotidique selon la revendication 1 ou 2.

6. Vaccin pour lever des anticorps contre une sous-unité β de la ganadotropine chorionique comprenant une composition pharmaceutique selon la revendication 5.
